# EUROPÄISCHE PATENTANMELDUNG

(11) **EP 4 649 983 A1**
(43) Veröffentlichungstag der Anmeldung: **19.11.2025**
(21) Anmeldenummer: 25173122.0
(22) Anmeldetag: 29.04.2025
(51) Int. Cl.: A61M 16/00, A61M 16/16, G10K 11/16

(54) **VORRICHTUNG ZUR SCHALLDÄMPFUNG FÜR EIN BEATMUNGSGERÄT**

(30) Priorität: 14.05.2024 DE 102024113434
(71) Anmelder: Löwenstein Medical Technology S.A., 2557 Luxembourg (LU)
(72) Erfinder: Gerlach, Angela, 22549 Hamburg (DE); Veers, Kathleen, 24116 Kiel (DE); Oster, Alexander, 24214 Gettorf (DE)
(74) Vertreter: Löwenstein Medical IP

(57) **Zusammenfassung**

Die Erfindung betrifft eine Vorrichtung zur Schalldämpfung für ein Beatmungsgerät sowie ein Beatmungsgerät. Das Beatmungsgerät umfasst neben der Vorrichtung einen Atemgaseingang, einen Atemgasausgang und einen zwischen dem Atemgaseingang und dem Atemgasausgang ausgebildeten Atemgasweg mit einem Gebläse, wobei das Gebläse ausgebildet ist, um ein Atemgas vom Atemgaseingang zum Atemgasausgang zu fördern, so dass der Atemgasweg eine Saugseite und eine Druckseite aufweist, wobei die Vorrichtung umfasst: eine Kammer, die ausgebildet ist, um zwischen dem Gebläse und dem Atemgasausgang auf der Druckseite im Atemgasweg angeordnet zu werden, und einen rohrförmigen Kanal, der ausgebildet ist, um die Kammer fluidisch mit dem Atemgasausgang zu verbinden.

## Beschreibung

### Technisches Gebiet

Die Erfindung betrifft eine Vorrichtung zur Schalldämpfung. Diese Vorrichtung ist für ein Beatmungsgerät ausgebildet. Des Weiteren betrifft die Erfindung ein Beatmungsgerät umfassend eine Vorrichtung zur Schalldämpfung.

### Stand der Technik

Ein Beatmungsgerät ist eine Vorrichtung zur Beatmung eines Individuums mit unzureichender, gestörter oder ausgesetzter Eigenatmung und kann sowohl im häuslichen Bereich als auch im klinischen Bereich Anwendung finden. Ein Beatmungsgerät kann bei der natürlichen Atmung unterstützen und/oder die Beatmung übernehmen. Beatmungsgeräte können auch anderweitig auf die Atmung eines Individuums einwirken, beispielsweise im Rahmen einer Atemtherapie, einer Sauerstoffversorgung, oder zur Hustenunterstützung. Darüber hinaus können Beatmungsgeräte zu Diagnosezwecken verwendet werden.

Ein Beatmungsgerät weist in der Regel zumindest einen Atemgasweg mit einem Eingang, einem Ausgang und einem Atemgasantrieb (Gebläse) zur Beförderung eines Atemgases auf. Damit das Beatmungsgerät nicht als störend empfunden wird, sollten die Betriebsgeräusche möglichst gering sein. Die Betriebsgeräusche entstehen durch Schallwellen, die beispielsweise vom Gebläse, von der Luftdurchführung und von Kühllüftern erzeugt werden. Aus dem Stand der Technik sind Beatmungsgeräte bekannt, die zur Verminderung der Betriebsgeräusche mit schalldämmenden Schäumen ausgestattet sind. Diese sind beispielsweise aus Polyester-basiertem Polyurethan ausgebildet. EP3708208B1 zeigt beispielsweise eine Vorrichtung zur Beatmung mit Absorberschäumen zur Schalldämmung. Es hat sich jedoch herausgestellt, dass sich einige Schäume negativ auf die Gesundheit der Patienten auswirken können.

### Offenbarung der Erfindung

Eine Aufgabe der vorliegenden Erfindung kann darin gesehen werden, eine Vorrichtung für ein Beatmungsgerät bereitzustellen, die eine effektive Schallunterdrückung bietet und einen sicheren und effizienten Betrieb des Beatmungsgerätes gewährleistet.

Diese Aufgabe wird gelöst mit einer Vorrichtung zur Schalldämpfung sowie mit einem Beatmungsgerät gemäß den unabhängigen Ansprüchen. Weiterbildungen und vorteilhafte Ausgestaltungen sind Gegenstand der Unteransprüche, deren Merkmale im Rahmen des technisch sinnvollen beliebig miteinander kombiniert werden können. Dies gilt insbesondere auch über die Grenzen der verschiedenen Anspruchskategorien hinaus. Die Beschreibung charakterisiert und spezifiziert die Erfindung insbesondere im Zusammenhang mit den Figuren zusätzlich.

Die Erfindung betrifft eine Vorrichtung zur Schalldämpfung für ein Beatmungsgerät. Das Beatmungsgerät umfasst neben der Vorrichtung einen Atemgaseingang, einen Atemgasausgang und einen zwischen dem Atemgaseingang und dem Atemgasausgang ausgebildeten Atemgasweg mit einem Gebläse, wobei das Gebläse ausgebildet ist, um ein Atemgas vom Atemgaseingang zum Atemgasausgang zu fördern, so dass der Atemgasweg eine Saugseite und eine Druckseite aufweist. Die Vorrichtung umfasst eine Kammer, die ausgebildet ist, um zwischen dem Gebläse und dem Atemgasausgang auf der Druckseite im Atemgasweg angeordnet zu werden, und einen rohrförmigen Kanal, der ausgebildet ist, um die Kammer fluidisch mit dem Atemgasausgang zu verbinden.

Unter "Beatmungsgerät" kann eine Vorrichtung zur Beatmung und/oder zur Narkotisierung verstanden werden. Das Beatmungsgerät kann somit eine Beatmungsfunktion und eine Anästhesiefunktion und eine Kombination aus diesen aufweisen. Unter "Beatmung" kann eine künstliche Beatmung, eine Atemunterstützung, eine Atemtherapie, eine Atemdiagnose, eine Hustenunterstützung, eine Sauerstoffliefernde Therapie wie beispielweise eine High-Flow-Therapie, eine Inhalationsnarkose und eine Kombination aus mindestens zwei dieser Beispiele verstanden werden. Somit kann ein Individuum unter Verwendung des Beatmungsgerätes beatmet oder in der Atmung unterstützt werden und alternativ oder zusätzlich auch unter Narkose gehalten werden. Zu diesem Zwecke kann das Beatmungsgerät auch mit Anästhesiegasen betrieben werden und somit einer Inhalationsanästhesie dienen.

Unter einem Beatmungsgerät können somit sämtliche Geräte verstanden werden, die ein Individuum bei der natürlichen Atmung unterstützen und/oder die Beatmung übernehmen und/oder einer Atemtherapie dienen und/oder einer Inhalationsnarkose dienen und/oder anderweitig auf die Atmung des Patienten oder Anwenders einwirken. Ein Beatmungsgerät kann zum Beispiel ein Beatmungsgerät für klinische oder Heimanwendungen, ein Atemtherapiegerät, ein CPAP-, APAP- oder BiLevel-Gerät, ein Highflow-Therapiegerät, ein Narkose- bzw. Anästhesiegerät, ein Notfall-Beatmungsgerät, ein Sauerstofflieferndes Gerät, ein Diagnosesystem oder ein Hustentherapiegerät bzw. eine Hustenmaschine sein.

Das Beatmungsgerät kann ein Gehäuse aufweisen. Der Atemgaseingang und der Atemgasausgang des Beatmungsgerätes können am Gehäuse angeordnet sein und/oder durch das Gehäuse ausgebildet sein. Zwischen dem Atemgaseingang und dem Atemgasausgang ist der Atemgasweg ausgebildet, der sich in der Regel - zumindest größtenteils - innerhalb des Gehäuses befindet. Der Atemgasweg ist eingerichtet und ausgebildet ein Atemgas zu führen. Unter "Atemgas" kann ein einzuatmendes und/oder ausgeatmetes Gas oder Gasgemisch verstanden werden. Das Atemgas kann beispielsweise normale Atemluft aus der Umgebung, Sauerstoff oder mit Sauerstoff angereicherte Atemluft sein. Unter Atemgas kann auch ein Gasgemisch verstanden werden, das mit mindestens einem Arznei- oder Narkosemittel angereichert ist, so dass das Atemgas zu Therapie- oder Narkosezwecken eingesetzt werden kann.

Der Atemgasweg kann mindestens einen Atemgasantrieb umfassen, um das Atemgas in mindestens einer Richtung zu führen. Der Atemgasantrieb kann beispielsweise als Gebläse, insbesondere als Radialgebläse oder als Axialgebläse, als Kolbenmotor, als Faltenbalg oder als Ventilanordnung ausgeführt sein. Vorzugsweise ist der Atemgasantrieb als Radialgebläse ausgeführt. Das Gebläse kann einen Saugstutzen und einen Druckstutzen aufweisen. Bei Radialgebläsen kann das Atemgas über den Saugstutzen in Achsrichtung in das Gebläse eintreten und über den Druckstutzen wieder aus dem Gebläse austreten.

In manchen Ausführungsformen kann das Atemgas über den Druckstutzen senkrecht zur Achsrichtung wieder aus dem Gebläse austreten. In manchen Ausführungsformen kann das Atemgas im Druckstutzen und/oder in Strömungsrichtung nach dem Druckstutzen auch umgelenkt werden. Somit kann das Atemgas auch in Radialgebläsen in Achsrichtung in das Gebläse eintreten, radial durch das Laufrad geführt und nach einer Umlenkung wieder axial aus dem Gebläse austreten. Zu diesem Zwecke kann der Druckstutzen eine Krümmung aufweisen und/oder das Gebläse kann Leitschaufeln aufweisen, die eingerichtet sind, um das Atemgas umzulenken.

Das Gebläse umfasst üblicherweise wenigstens ein drehbar gelagertes Laufrad, das von einem Motor angetrieben wird und eine Umlenkung des Atemgases von axial nach radial erzeugt. Dabei wird eine Atemgas-Strömung erzeugt. Für eine Beatmung kann das Gebläse Drehzahlen von bis zu 60.000 U/min oder sogar darüber hinaus leisten, so dass ein entsprechender Atemgas-Druck aufgebaut werden kann.

Durch das Gebläse kann der Atemgasweg in eine Saugseite und eine Druckseite unterteilt sein. Die Saugseite befindet sich stromaufwärts vor dem Saugstutzen des Gebläses. Das Atemgas kann über den Atemgaseingang in den Atemgasweg eingespeist werden und saugseitig zum Gebläse gefördert werden. Die Saugseite befindet sich somit zwischen dem Atemgaseingang und dem Saugstutzen des Gebläses. Die Druckseite befindet sich stromabwärts nach dem Druckstutzen des Gebläses. Über die Druckseite und den Atemgasausgang kann das Atemgas vom Gebläse zum Patienten befördert werden. Die Druckseite befindet sich somit zwischen dem Druckstutzen des Gebläses und dem Atemgasausgang.

Das Beatmungsgerät kann auch über mehrere Eingänge verfügen, über die alternativ oder zusätzlich weitere Atemgase in den Atemgasweg eingeleitet werden können wie beispielsweise Frischgas, Sauerstoff, Gasgemische, Arznei- oder Narkosemittel. Alternativ oder zusätzlich kann das Beatmungsgerät auch über mehrere Ausgänge verfügen. Der Atemgaseingang und/oder der Atemgasausgang kann einen Anschluss ausbilden oder umfassen, um jeweils einen Schlauch oder ein Schlauchsystems oder ein Patienteninterface auszuschließen.

Über den Atemgasausgang kann das Atemgas aus dem Atemgasweg entlassen werden und dem Patienten beispielsweise über den Schlauch oder das Schlauchsystem und/oder über das Patienteninterface bereitgestellt werden. Unter "Patienteninterface" kann im Sinne der Erfindung jegliches Peripheriegerät verstanden werden, welches zur Interaktion mit einem Lebewesen ausgebildet ist. Insbesondere ist das Patienteninterface zu Beatmungs-, Therapie- und/oder Diagnosezwecken in Verbindung mit dem Beatmungsgerät ausgebildet. Das Patienteninterface kann als Atemmaske ausgebildet sein. Darunter fallen zum Beispiel, aber nicht ausschließlich, Nasenmasken, Nasenpolstermasken, Nasenbrillen bzw. Sauerstoffbrillen, Full-Face- oder Totalface-Masken sowie Trachealtuben bzw. -kanülen.

Beim Betrieb des Beatmungsgerätes können Betriebsgeräusche entstehen, die beispielsweise vom Gebläse, vom Laufrad, vom Motor, von der Luftdurchführung oder von Kühllüftern erzeugt werden. Für den Patienten kann es als besonders störend empfunden werden, wenn die Betriebsgeräusche druckseitig entstehen oder vorhanden sind und somit direkt zum Patienten gelangen können. Mit der vorliegenden Erfindung kann insbesondere der druckseitige Schall gedämpft werden.

Unter "Schalldämpfung" kann im Sinne der Erfindung allgemein verstanden werden, dass Schall reduziert wird. Die Reduktion des Schalls kann durch Dämpfung und/oder Dämmung und/oder akustischen Impedanzen und/oder Reflexionen und/oder Schallwellen-Überlagerung (Interferenz) und/oder Absorption erfolgen. Der Begriff "Schalldämpfung" wird hierin als Überbegriff verwendet für sämtliche physikalischen Effekte, die den Schall reduzieren können.

Mit der vorliegenden Erfindung wird der Schall insbesondere durch akustische Impedanzen gedämpft. Eine "akustische Impedanz", insbesondere eine akustische Flussimpedanz, benennt den Widerstand, der einer Schallausbreitung, zum Beispiel in Rohren, entgegengesetzt wird. Bei einem Übergang zwischen Bereichen mit unterschiedlicher akustischer Impedanz kommt es zu Reflexionen der Schallwellen, wodurch eine schalldämpfende Wirkung erreicht wird.

Die Vorrichtung gemäß der vorliegenden Offenbarung mit der Kammer und dem Kanal führt zu einer deutlichen Verringerung der von einem Beatmungsgerät ausgehenden Betriebsgeräusche. Da die Kammer und der Kanal auf der Druckseite des Atemgasweges eines Beatmungsgerätes angeordnet werden können, kann insbesondere der beim Patienten ankommende druckseitige Schall gedämpft werden.

Unter "Kanal" kann beispielsweise ein Rohr, ein Schlauch oder eine Kombination aus beidem verstanden werden. Der Kanal kann starr oder zumindest teilweise flexibel ausgebildet sein. Der Kanal ist als länglicher Hohlraum mit einer ein Lumen des Kanals umschließenden Wandung ausgebildet. Der Kanal weist längsseitig zwei Öffnungen auf, durch die Atemgas in den Kanal ein- oder austreten kann.

Der Kanal ist ausgebildet, um ein Atemgas aufzunehmen und/oder zu leiten. Der Kanal weist eine Länge, eine Höhe und eine Breite auf. In der Regel ist die Länge des Kanals um ein Vielfaches größer als die Breite und/oder die Höhe. Höhe und Breite des Kanals können gleich sein oder voneinander (geringfügig) abweichen. Im Lumen des Kanals kann das Atemgas aufgenommen und/oder geleitet werden. Das Atemgas kann - angetrieben von dem Atemgasantrieb des Beatmungsgerätes -durch den Kanal geleitet werden. Dabei entspricht die Durchflussrichtung in der Regel der Längsrichtung des Kanals. Orthogonal zur Durchflussrichtung weist der Kanal einen Querschnitt auf.

Unter "Kammer" kann ein Bereich verstanden werden, der in Relation zum Kanal eine abweichende Geometrie aufweist. Die Kammer ist ebenso wie der Kanal ausgebildet, um ein Atemgas aufzunehmen und/oder zu leiten. Die Kammer ist als Hohlraum mit einer ein Lumen der Kammer umschließenden Wandung ausgebildet. Die Kammer kann zwei Öffnungen aufweisen, durch die Atemgas in die Kammer ein- oder austreten kann. Die Kammer kann eine Kammerwand aufweisen, dessen Innenwandung ein Lumen der Kammer umschließt. Im Lumen der Kammer kann das Atemgas aufgenommen bzw. geleitet werden. Die Kammer kann auch, zumindest in Teilen, durch das Beatmungsgerät selbst gebildet sein, beispielsweise durch Teile des Gehäuses, Außenwände anderer Komponenten des Beatmungsgerätes und dergleichen. Die Kammer weist eine Länge, eine Höhe und eine Breite auf. Im Gegensatz zum Kanal ist die Länge in der Regel nicht um ein Vielfaches größer als die Breite und/oder die Höhe. Orthogonal zur Durchflussrichtung weist die Kammer einen Querschnitt auf. Der Querschnitt der Kammer ist in der Regel größer als der Querschnitt des Kanals.

Der Atemgasweg weist somit unterschiedliche Abschnitte auf. Das Atemgas strömt saugseitig vom Atemgaseingang zum Gebläse und druckseitig vom Gebläse zum Atemgasausgang. Das Gebläse kann als ein Abschnitt des Atemgasweges verstanden werden. Auf der Saugseite und/oder auf der Druckseite können weitere Abschnitte angeordnet sein, insbesondere Kanäle und Kammern mit unterschiedlichen Geometrien und Abmessungen. Vorzugsweise sind auf der Druckseite ein oder mehrere Kanäle und/oder Kammern ausgebildet. Eine Anordnung von Kammern und/oder Kanälen auf der Saugseite ist in manchen Ausführungsformen ebenfalls denkbar.

In Ausgestaltung kann die Vorrichtung einen modularen Aufbau haben. Ein Modul kann dabei einem Abschnitt entsprechen. Dementsprechend kann ein Modul eine Kammer umfassen, ein weiteres einen Kanal usw. Die Module können derart ausgebildet sein, dass sie in unterschiedlicher Reihung hintereinander angeordnet werden können. Die Module können beispielsweise als Stecksystem ausgebildet sein, so dass eine beliebige Reihenfolge der Module realisiert werden kann. Dies bietet den Vorteil eines besonders flexiblen Systems. Die Module können derart angeordnet sein, dass sich in Strömungsrichtung nach dem Druckstutzen eine Kammer anschließt und nach der Kammer ein Kanal. Zusätzliche Kanäle und Kammern können folgen.

Der Atemgasweg erfährt durch die unterschiedlichen Abschnitte (Module) mindestens eine, vorzugsweise mehrere, Querschnittsänderung und/oder Formänderung. Die geometrische Form des Atemgasweges wird zumindest einmal, vorzugsweise mehrmals, geändert. Dadurch kann der Querschnitt des Atemgasweges zumindest einmal, vorzugsweise mehrmals, verkleinert oder vergrößert werden. Dabei ist es bevorzugt, wenn sich der Querschnitt des Atemgaswegs an einem Übergang zwischen zwei Abschnitten (Gebläse, Kammer, Kanal) abrupt oder sprunghaft verkleinert oder vergrößert. In einer bevorzugten Ausführungsform ist der Übergang zwischen den Abschnitten abrupt, so dass eine deutliche geometrische Diskontinuität auftritt.

In Ausgestaltung kann der Querschnitt des Gebläses, insbesondere der Querschnitt des Druckstutzens, kleiner als der Querschnitt der Kammer sein. Der Querschnitt der Kammer kann wiederrum größer als der Querschnitt des Kanals sein. Die Querschnitte des Druckstutzens und des Kanals können gleich sein oder bevorzugt unterschiedlich.

Um eine Schalldämpfung zu erreichen ist mindestens ein Querschnittssprung nötig, bevorzugt zwei, besonders bevorzugt drei oder mehr. Je mehr Querschnittssprünge in dem Atemgasweg vorhanden sind, desto größer ist die schalldämpfende Wirkung. Dabei sollte jedoch beachtet werden, dass die Vorrichtung in Ausgestaltung so klein wie möglich konstruiert werden sollte, da die unterschiedlichen Abschnitte und/oder Querschnitte einen gewissen Druckverlust mit sich bringen. Um diesen auszugleichen, können die Drehzahlen des Gebläses angepasst werden.

Gemäß einer Ausführungsform kann das Gebläse eine erste akustische Impedanz aufweisen und die Kammer kann eine zweite akustische Impedanz aufweisen und der Kanal kann eine dritte akustische Impedanz aufweisen, wobei die akustischen Impedanzen derart voneinander abweichen, dass ein durch das Gebläse erzeugter Schall gedämpft wird.

Die Vorrichtung ist derart ausgebildet, dass bevorzugt eine sprunghafte Änderung der Impedanzen zwischen dem Gebläse und der Kammer bestehen. Alternativ oder zusätzlich kann eine sprunghafte Änderung der Impedanzen zwischen der Kammer und dem Kanal bestehen. Alternativ oder zusätzlich kann auch eine sprunghafte Änderung der Impedanzen zwischen dem Gebläse und dem Kanal bestehen. Dies wird wie oben beschrieben durch unterschiedliche Geometrien und/oder Querschnitte erzielt.

Gemäß einer Ausführungsform kann der Kanal eine Kanalwand mit einer ein Lumen des Kanals umschließenden Innenwandung umfassen. Die Innenwandung des Kanals kann konstant ausgebildet sein. Alternativ oder zusätzlich kann die Kammer eine Kammerwand mit einer ein Lumen der Kammer umschließenden Kammer-Innenwandung umfassen. Die Kammer-Innenwandung kann konstant ausgebildet sein.

Unter "konstant" kann verstanden werden, dass die Kanalwand und/oder die Kammerwand ohne eine Veränderung oder einen Bruch fortdauernd einheitlich besteht. Eine konstante Wand ist lückenlos zusammenhängend und setzt sich - zumindest innerhalb eines Abschnittes - gleichmäßig fort.

In manchen Ausführungsformen kann die Kanalwand und/oder die Kammerwand auch, zumindest in Teilen, durch das Beatmungsgerät selbst ausgebildet sein. So können beispielsweise Teile des Gehäuses, Außenwände anderer Komponenten des Beatmungsgerätes und dergleichen die Kammerwand oder die Kanalwand zumindest teilweise ausbilden. Es hat sich jedoch als vorteilhaft erwiesen, dass Kammerwand und Kanalwand konstant, bestenfalls aus einer eigenen Wandung bestehen. Kanal und Kammer können sich zumindest abschnittweise die Wandung teilen. Das kann bedeuten, dass die Kanalwand in einem Betriebszustand der Vorrichtung, also in einem Zustand, in denen die Module aneinander angrenzen, gleichzeitig auch die Begrenzung der Kammer ausbildet und vice versa.

Gemäß einer Ausführungsform kann die Vorrichtung eine zweite Kammer umfassen, die zwischen dem Kanal und dem Atemgasausgang angeordnet ist. Die zweite Kammer kann ausgebildet sein, um eine weitere geometrische Form und/oder eine weitere Querschnittsänderung in den Atemgasweg einzuführen. Vorzugsweise ist die zweite Kammer eingerichtet, um eine sprunghafte Vergrößerung oder Verkleinerung des Querschnittes des Atemgasweges auszubilden. Die zweite Kammer kann einen weiteren Querschnittssprung des Atemgasweges verursachen, so dass die schalldämpfende Wirkung der Vorrichtung verstärkt wird. Die zweite Kammer kann entsprechend der ersten Kammer eine Kammerwand mit einer ein Lumen der Kammer umschließenden Kammer-Innenwandung umfassen. Die Kammer-Innenwandung der zweiten Kammer kann konstant ausgebildet sein.

Gemäß einer Ausführungsform kann die zweite Kammer eine vierte akustische Impedanz aufweisen, die von der ersten akustischen Impedanz und/oder der zweiten akustischen Impedanz und/oder der dritten akustischen Impedanz abweicht, sodass ein durch das Gebläse erzeugter Schall zusätzlich gedämpft wird. Die zweite Kammer kann derart ausgebildet sein, dass eine sprunghafte Änderung der Impedanzen zwischen der zweiten Kammer und dem Kanal und/oder der Kammer und/oder dem Gebläse besteht. Somit wird eine weitere Schalldämpfung erreicht.

Gemäß einer Ausführungsform kann der Kanal und/oder die Kammer und/oder die zweite Kammer als Hohlraum in einem nicht-porösem Material ausgebildet ist, insbesondere in einen nicht porösen Kunststoff. Das bedeutet, dass die Kanalwand und/oder die Kammerwand ein nicht-poröses Material umfassen. Bevorzugt sind zumindest die Kanal-Innenwandung und die Kammer-Innenwandungen aus einem nicht-porösen Material gefertigt.

Unter "nicht-porös" kann verstanden werden, dass das Material ohne (gewollte) Lufteinschlüsse, Poren oder dergleichen ausgebildet ist. Ein nicht-poröses Material weist optimalerweise ausschließlich Merkmale eines festen Aggregatzustandes auf. Das Material kann beispielsweise ein Metall und/oder ein nicht-poröses Polymermaterial (Kunststoff) sein oder umfassen. Die Vorrichtung kann beispielsweise mittels Gießen, Spritzgießen oder dergleichen hergestellt sein.

Das Material kann mindestens eines der folgenden metallischen Werkstoffe umfassen: Eisen; Stahl, insbesondere Edelstahl; Aluminium; Kupfer oder andere geeignete metallische Werkstoffe. Das Material kann alternativ oder zusätzlich mindestens eines der folgenden polymeren Werkstoffe umfassen: Silikon, Naturkautschuk, Polyvenylchlorid (PVC), Polymethylmethacrylat (PMMA) oder andere geeignete Kunststoffe.

Die metallischen Werkstoffe können eine Dichte von 2.000 bis 10.000 kg/m³ aufweisen, bevorzugt von 2700 bis 8950 kg/m³. Die metallischen Werkstoffe können eine Härte von 20 bis 120 HB aufweisen. Die metallischen Werkstoffe können eine Zugfestigkeit von 60 bis 950 N/mm² aufweisen.

Die polymeren Werkstoffe können eine Dichte von 800 bis 1.500 kg/m³ aufweisen, bevorzugt von 920 bis 1.400 kg/m³. Die polymeren Werkstoffe können eine Shore A Härte von 20 bis 100 Shore A aufweisen. Die polymeren Werkstoffe können eine Zugfestigkeit von 1 bis 80 N/mm² aufweisen.

Es hat sich als besonders vorteilhaft erwiesen, dass der Atemgasweg im Wesentlichen ohne Schaumstoff und/oder ohne Flies ausgebildet ist. Dadurch kann der Atemgasweg für den Patienten besonders sicher ausgebildet sein, da keine gesundheitsschädlichen Substanzen im Atemgasweg gebildet und/oder an das Atemgas abgegeben werden können. Ferner ist eine Reinigung, Desinfektion oder Sterilisation vereinfacht und sicherer, wenn der Atemgasweg aus einem nicht-porösen Material ausgebildet ist, da das nicht-poröse Material nicht in dem Maße von den Reinigungsmitteln angegriffen wird wie Schaumstoffe.

Vorzugsweise kann insbesondere der Kanal und/oder die Kammer und/oder die zweite Kammer, weiter vorzugsweise alle Komponenten des Beatmungsgeräts entlang des Atemgasweges, ohne Schaumstoff und/oder Flies ausgebildet sein.

Allerdings kann stromaufwärts des Atemgasweges ein Filter, beispielsweise ein Faltenfilter, oder ein Filtersystem aus mehreren hintereinander angeordneten Filtern und/oder ein Flies angeordnet sein. Der Filter und/oder das Flies kann im oder am Atemgaseingang des Beatmungsgerätes angeordnet sein. Der Filter und/oder das Flies kann austauschbar sein. Der Filter und/oder das Flies kann eingerichtet sein, um ein über den Atemgaseingang in den Atemgasweg einströmendes Atemgas zu filtern. Der Filter und/oder das Flies kann eingerichtet sein, um mögliche Feststoff-Partikel aus dem Atemgas herauszufiltern.

Gemäß einer Ausführungsform kann der Kanal orthogonal zu einer Längsrichtung des Kanals einen Querschnitt aufweisen, der rund oder eckig ist. Der Kanal kann einen beliebigen Querschnitt aufweisen. Der Querschnitt kann gemäß vorliegender Offenbarung rund oder eckig sein, beispielsweise kreisrund, oval, viereckig, rechteckig oder mehreckig. Andere Querschnitte sind jedoch ebenfalls möglich. Der Querschnitt des Kanals kann zumindest abschnittweise beispielsweise auch schlitzförmig, sichelförmig, Kreuz-förmig, Stern-förmig, Trapez-förmig, X-förmig, L-förmig, U-förmig, V-förmig, T-förmig, Ellipsen-förmig, oder dergleichen sein. Der Querschnitt kann konstant sein oder sich im Laufe der Längsrichtung ändern. Beispielsweise kann der Querschnitt in manchen Ausführungsformen seine geometrische Form verändern oder in seinem Umfang zunehmen und/oder abnehmen. Vorzugsweise weist der Kanal einen runden oder ovalen Querschnitt auf, da ein runder bzw. ovaler Querschnitt die besten Strömungseigenschaften aufweist.

In einer bevorzugten Ausführungsform bleibt der Querschnitt im Verlaufe der Längsrichtung des Kanals konstant. Es ist in manchen Ausführungsformen jedoch auch denkbar, dass der Kanal im Verlauf seiner Längsrichtung zunimmt und/oder abnimmt.

Gemäß einer Ausführungsform kann der Kanal in seiner Längsrichtung betrachtet zumindest teilweise gerade und/oder zumindest teilweise gebogen ausgebildet sein. In einer einfachen Ausführungsform ist der Kanal in seiner Längsrichtung gerade ausgebildet. Es kann jedoch vorteilhaft sein, wenn der Kanal zumindest teilweise gebogen ausgebildet ist. Somit kann die Vorrichtung besonders platzsparend ausgebildet sein. Ferner kann ein gebogener Kanal eine vorteilhafte Umlenkung des Atemgases bewirken, die sich positiv auf die Schallunterdrückung auswirken kann.

Gemäß einer Ausführungsform kann der Kanal mindestens einen geraden Abschnitt und mindestens einen gebogenen Abschnitt umfassen, wobei der gebogene Abschnitt mindestens eine Biegung aufweist, so dass der Kanal in seiner Längsrichtung gebogen ist. Gemäß einer Ausführungsform kann die Biegung rund oder eckig ausgebildet sein. Die Biegung kann mindestens 10° betragen, bevorzugt mindestens 45°. Die Biegung kann besonders bevorzugt zwischen 45° und 180° betragen. Gemäß einer Ausführungsform kann der Kanal in seiner Längsrichtung an mindestens einer Stelle U-förmig und/oder L-förmig gebogen sein, bevorzugt an mindestens zwei Stellen. Durch eine U-förmige Biegung kann der Kanal an mindestens einer Stelle um 180° gebogen sein. Durch eine L-förmige Biegung kann der Kanal an mindestens einer Stelle um 90° gebogen sein. Die Biegung(en) können rund oder eckig ausgebildet sein.

Gemäß einer Ausführungsform kann der Kanal einen ersten geraden Abschnitt, einen zweiten geraden Abschnitt und einen den ersten geraden Abschnitt mit dem zweiten geraden Abschnitt verbindenden gebogenen Abschnitt umfassen, wobei der gebogene Abschnitt eine U-förmige Biegung aufweist, so dass die Längsachsen des ersten geraden Abschnitts und des zweiten geraden Abschnitts parallel zueinander sind. Diese Ausführungsform kann besonders platzsparend ausgebildet sein.

Gemäß einer Ausführungsform kann der Kanal einen ersten gebogenen Abschnitt und einen zweiten gebogenen Abschnitt und einen den ersten gebogenen Abschnitt mit dem zweiten gebogenen Abschnitt verbindenden geraden Abschnitt umfassen.

Gemäß einer Ausführungsform kann der Kanal eine Mehrzahl an geraden Abschnitten und gebogenen Abschnitten umfassen, so dass der Kanal in seiner Längsrichtung zumindest abschnittsweise einen mäanderförmigen Verlauf aufweist. Unter "mäanderförmig" kann verstanden werden, dass der Kanal eine Abfolge an mehreren Biegungen aufweist. Diese können regelmäßig oder unregelmäßig angeordnet sein, so dass der Kanal symmetrisch oder asymmetrisch ausgebildet sein kann. Ein mäanderförmiger Verlauf bietet den Vorteil einer besonderen Platzersparnis bei einer möglichst großen Länge des Kanals. Die Biegungen können in einer Ebene verlaufen und den Kanal somit zweidimensional biegen. Es ist in manchen Ausführungsformen auch möglich, dass der Kanal in einer weiteren Ebene gebogen ist, so dass eine oder mehrere dreidimensionale Biegung(en) vorliegen. Die Biegungen des Kanals können sich in jede erdenkliche Richtung erstrecken. Durch die Biegungen des Kanals kann der Kanal symmetrisch oder asymmetrisch ausgebildet sein. Der Kanal kann eine beliebige räumliche Anordnung aufweisen. Es ist allerdings vorteilhaft, wenn der Kanal ein bestimmtes Volumen und/oder bestimmte Abmessungen nicht über- oder unterschreitet.

Gemäß einer Ausführungsform kann der Kanal eine Mehrzahl an geraden Abschnitten und gebogenen Abschnitten umfassen und derart angeordnet sein, dass der Kanal zumindest teilweise die erste Kammer ausbildet, indem eine Wand des Kanals zumindest bereichsweise eine Wand der Kammer ausbildet.

Gemäß einer Ausführungsform kann der Kanal um die Kammer herum U-förmig angeordnet sein. Es kann auch vorgesehen sein, dass der Kanal nur gebogene Abschnitte umfasst, so dass dieser oval, kreisrund oder spiralförmig ausgebildet ist. Optional kann der derartig ausgebildete Kanal um die Kammer herumgewickelt ausgebildet sein. Dies kann eine besonders platzsparende Ausführungsform darstellen.

Gemäß einer Ausführungsform kann der Kanal ein Volumen von 100 cm³ - 10.000 cm³ aufweisen, bevorzugt von 500 cm³ - 5.000 cm³. Es hat sich als besonders vorteilhaft erwiesen, dass der Kanal ein Volumen von 800 - 4.500 cm³ aufweist. Um dieses Volumen zu erreichen, weist der Kanal eine Länge und einen Durchmesser auf. Gemäß einer Ausführungsform kann der Kanal eine Länge von1 mm - 1.000 mm aufweisen, bevorzugt 10 mm - 500 mm. Als besonders vorteilhaft hat sich eine Länge von 10 - 350 mm erwiesen. Ferner kann der Kanal einen Durchmesser von1 mm - 30 mm, bevorzugt 5 mm - 20 mm, besonders bevorzugt 10 - 15 mm aufweisen. Die Länge des Kanals bedingt eine deutlich verminderte druckseitige Schallabstrahlung.

Gemäß einer Ausführungsform kann die Kammer und/oder die zweite Kammer ein Volumen von 2.000 cm³ - 40.000 cm³ aufweisen, bevorzugt von 4.000 cm³ - 20.000 cm³, besonders bevorzugt von 10.000 - 15.000 cm³. Die Schalldämpfende Wirkung tritt insbesondere dann ein, wenn die Kammer bzw. die zweite Kammer wie voranstehend definiert dimensioniert ist. Gemäß einer Ausführungsform kann die Kammer und/oder die zweite Kammer eine Tiefe von 50 mm - 120 mm aufweisen, bevorzugt von 80 mm - 100 mm und/oder eine Höhe von 10 mm - 70 mm, bevorzugt von 20 mm - 40 mm und/oder eine Breite von 10 mm - 100 mm, bevorzugt von 40 mm - 60 mm.

Die Tiefe der Kammern ist als eine Ausdehnung der Kammervolumina in Strömungsrichtung zu verstehen. Die Breite und die Höhe der Kammern definieren einen Querschnitt der Kammern, der senkrecht zur Tiefe, und somit der Strömungsrichtung des Atemgases, ausgerichtet ist.

Der Querschnitt der Kammer und der zweiten Kammer kann eine beliebige geometrische Form oder Querschnitt aufweisen. Sie kann gemäß vorliegender Offenbarung eckig, insbesondere rechteckig sein. Sie muss jedoch nicht eckig sein und kann gemäß einer Ausführungsform der vorliegenden Offenbarung auch rund oder oval sein. Sowohl längliche als auch kurze Ausgestaltungsformen der Kammer sind gemäß vorliegender Offenbarung möglich.

Es hat sich allerdings als vorteilhaft herausgestellt, wenn die Kammer eine gewisse Tiefe aufweist, wie voranstehend definiert. Wenn die Tiefe zu klein gewählt ist, würde die einkommende Atemgas-Strömung direkt auf die Gegenwand der Kammer treffen, was zu Strömungsgeräuschen führen kann.

Gemäß einer Ausführungsform kann das Verhältnis des Volumens des Kanals zum Volumen der Kammer und/oder der zweiten Kammer mindestens 1:2 betragen, bevorzugt mindestens 1:4, besonders bevorzugt 1:10.

Gemäß einer Ausführungsform können die Kammer und die zweite Kammer identisch ausgebildet sein. Gemäß einer Ausführungsform können die Kammer und die zweite Kammer unterschiedlich dimensioniert sein, so dass die akustischen Impedanzen voneinander abweichen. Gemäß einer Ausführungsform können die zweite Kammer und der Kanal unterschiedlich dimensioniert sein, so dass die akustischen Impedanzen voneinander abweichen.

Unter "Dimensionierung" kann die geometrische Form und/oder die Größe und/oder das Volumen und/oder die Abmessungen der Länge/Höhe/Tiefe verstanden werden. So ist es beispielsweise denkbar, dass die Kammer und die zweite Kammer eine unterschiedliche Größe und somit unterschiedliche Volumina aufweisen. Die Kammer und die zweite Kammer können in manchen Ausführungsformen auch das gleiche Volumen aufweisen aber unterschiedliche Abmessungen der Länge und/oder Höhe und/oder Tiefe aufweisen. Es ist auch denkbar, dass die Kammer und der zweiten Kammer unterschiedliche geometrische Formen aufweisen, so dass die Kammer beispielsweise viereckig oder mehreckig ausgebildet ist und die zweite Kammer beispielsweise rund oder oval, und vice versa. Die geometrischen Formen können symmetrisch oder asymmetrisch ausgebildet sein. So ist beispielsweise auch denkbar, dass die Kammer symmetrisch ausgebildet ist und die zweite Kammer asymmetrisch, und vice versa.

Durch eine unterschiedliche Dimensionierung der beiden Kammern kann man diese gegeneinander verstimmen. Dies kann bedeuten, dass es keine Wellenkonfiguration gibt, die genau in die Kammer bzw. die zweite Kammer reinpasst. Somit können die Kammer und/oder die zweite Kammer eingerichtet sein, dass die Schallwellen, deren Länge genau in die Kammergeometrie passt, nicht durch die Kammern hindurchgehen können. Dadurch kann der Schall gedämpft werden.

Gemäß einer Ausführungsform kann die Vorrichtung einen ersten Anschluss und/oder einen zweiten Anschluss umfassen, um die Vorrichtung an den Atemgasweg eines Beatmungsgerätes anzuschließen. Der Atemgasweg kann ausgebildet sein, um ein Atemgas zu leiten. Das Atemgas kann innerhalb des Beatmungsgerätes folgendem Atemgasweg folgen: Das Atemgas kann über den Atemgaseingang in den Atemgasweg eintreten. Vom Atemgaseingang kann das Atemgas über den saugseitigen Abschnitt des Atemgasweges und den Saugstutzen in das Gebläse eintreten. Das Gebläse liefert die Förderenergie des Atemgases und befördert das Atemgas über den Druckstutzen in den druckseitigen Abschnitt des Atemgasweges. Dort tritt das Atemgas über den ersten Anschluss in die Vorrichtung ein. Zunächst gelangt das Atemgas in die Kammer und durchströmt diese. Von der Kammer tritt das Atemgas in den Kanal ein und durchströmt diesen in seiner Längsrichtung. Vom Kanal kann das Atemgas über den ersten Anschluss aus der Vorrichtung austreten. Sodann kann das Atemgas über den Atemgasausgang aus dem Beatmungsgerät heraus und zu einem nicht gezeigten Patienten befördert werden.

Das Beatmungsgerät kann einen Befeuchter umfassen, der eingerichtet ist, das Atemgas mit einer Flüssigkeit zu befeuchten und/oder zu erwärmen. Der Befeuchter kann zu diesem Zwecke ein Flüssigkeitsdepot umfassen. Ferner kann eine Heizvorrichtung zum Erwärmen des Wassers umfasst sein, beispielsweise in Form eines Heizstabs. Zudem kann eine Vorrichtung zur Temperaturmessung der Flüssigkeit umfasst sein, beispielsweise in Form eines Tauchrohrs. Der Befeuchter kann somit ausgebildet sein, um ein erwärmtes Wasser bereitzustellen, über das Atemgas geleitet und somit erwärmt und/oder befeuchtet werden kann.

In manchen Ausführungsformen kann der Befeuchter die Vorrichtung zur Schalldämpfung zumindest teilweise umfassen. So kann die Kammer und/oder die zweite Kammer und/oder den Kanal im Befeuchter angeordnet sein oder durch diesen ausgebildet sein.

Zwischen dem Gebläse und der Kammer und/oder zwischen der Kammer und dem Kanal und/oder zwischen dem Kanal und der zweiten Kammer bestehen jeweils Übergänge. Die Übergänge können als Öffnung in der Wandung mindestens eines der jeweiligen Elemente ausgebildet sein. So können die Elemente einfach aneinander angrenzen und das Atemgas kann von einem Element in das Nächste strömen. In manchen Ausführungsformen ist es auch denkbar, dass ein Element in das Nächste hineinragend ausgebildet ist.

Die Kammer kann beispielsweise unmittelbar an den Druckstutzen des Gebläses anschließen. So kann beispielsweise der Druckstutzen direkt an eine Öffnung in der Kammer-Wandung angrenzen. In manchen Ausführungsformen kann der Druckstutzen auch in die Wandung hinein oder durch die Wandung der Kammer hindurchgeführt sein und in die Kammer hineinragen. Vorzugweise befindet sich zwischen dem Gebläse und der Kammer kein zusätzliches Element (Kammer oder Kanal oder sonstiger Hohlraum).

Ähnlich kann es sich beim Übergang zwischen der Kammer bzw. der zweiten Kammer und dem Kanal verhalten. Der Kanal kann unmittelbar an die Kammer bzw. die zweite Kammer anschließen. In manchen Ausführungsformen kann der Kanal auch in die Wandung hinein oder durch die Wandung der Kammer bzw. der zweiten Kammer hindurchgeführt sein und in die Kammer hineinragen.

Die Erfindung betrifft zudem ein Beatmungsgerät. Das Beatmungsgerät weist einen Atemgaseingang, einen Atemgasausgang und einem zwischen dem Atemgaseingang und dem Atemgasausgang ausgebildeten Atemgasweg mit einem Gebläse auf, das ausgebildet ist, um ein Atemgas vom Atemgaseingang zum Atemgasausgang zu fördern, so dass der Atemgasweg eine Saugseite und eine Druckseite aufweist. Die Saugseite ist zwischen dem Atemgaseingang und einem Gebläseeingang ausgebildet und die Druckseite ist zwischen einem Gebläseausgang und dem Atemgasausgang ausgebildet. Das Beatmungsgerät umfasst eine zuvor beschriebene Vorrichtung zur Schalldämpfung, wobei die Vorrichtung in dem Atemgasweg zwischen dem Gebläseausgang und dem Atemgasausgang angeordnet ist und über den ersten Anschluss und den zweiten Anschluss an den Atemgasweg angeschlossen ist.

### Kurze Beschreibung der Figuren

Im Folgenden werden Ausführungsformen der Erfindung mit Bezug auf die beigefügten Figuren beschrieben. Die Erfindung ist nicht auf die dargestellten Ausführungsbeispiele beschränkt. Weder die Beschreibung noch die Figuren sind als Beschränkung des Umfangs der Erfindung zu verstehen.
Figur 1 zeigt eine schematische Darstellung eines Beatmungsgerätes 1 mit einer Vorrichtung 10 gemäß einer Ausführungsform der Erfindung.
Figur 2 zeigt die Vorrichtung 10 gemäß einer ersten Ausführungsform der Erfindung.
Figur 3 zeigt beispielhafte Ausführungsformen eines Kanal 13 gemäß der Erfindung.
Figur 4 zeigt die Vorrichtung 10 gemäß einer zweiten Ausführungsform der Erfindung.
Figur 5 zeigt die Vorrichtung 10 gemäß einer dritten Ausführungsform der Erfindung.
Figur 6 zeigt die Vorrichtung 10 gemäß einer vierten Ausführungsform der Erfindung.

Die Figuren sind rein schematisch und mitunter nicht maßstabsgetreu. Werden in verschiedenen Figuren gleiche Bezugszeichen verwendet, so bezeichnen diese Bezugszeichen gleiche oder gleichwirkende Merkmale.

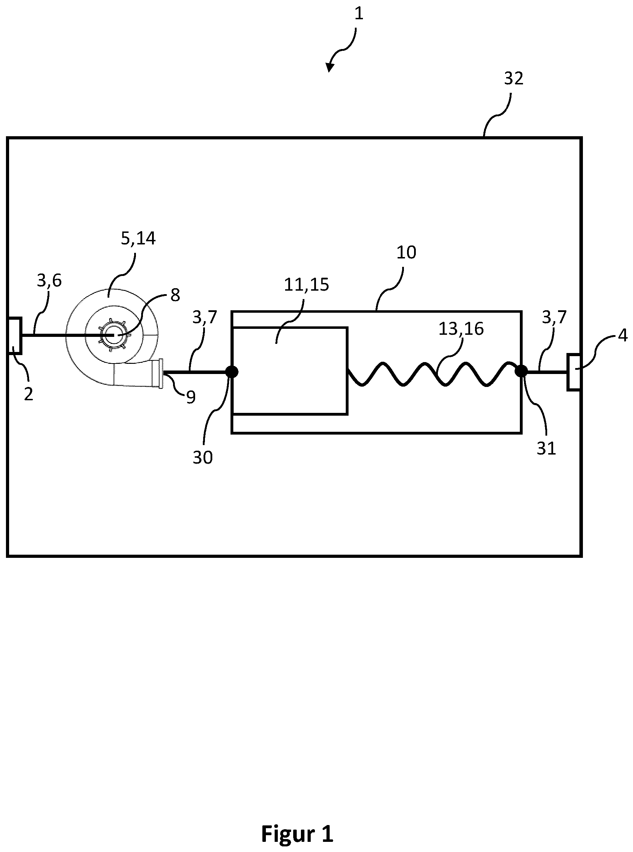

**Figur 1** zeigt eine schematische Darstellung eines Beatmungsgerätes 1 mit einer Vorrichtung 10 gemäß einer Ausführungsform der Erfindung. Das Beatmungsgerät 1 umfasst einen Atemgaseingang 2, einen Atemgasausgang 4 und einen zwischen dem Atemgaseingang 2 und dem Atemgasausgang 4 ausgebildeten Atemgasweg 3. Der Atemgasweg 3 ist ausgebildet, um ein Atemgas aufzunehmen und/oder zu führen und/oder zu leiten.

Das Beatmungsgerät 1 kann, wie schematisch angedeutet, ein Gehäuse 32 umfassen. Atemgaseingang 2 und Atemgasausgang 4 können am Gehäuse 32 angeordnet oder durch dieses ausgebildet sein. Es ist auch möglich, dass das Beatmungsgerät 1 mehrere Eingänge 2 und/oder Ausgänge 4 aufweist (nicht gezeigt). Atemgaseingang 2 und Atemgasausgang 4 können sich im Gehäuse 32 gegenüberliegen wie gezeigt. Atemgaseingang 2 und Atemgasausgang 4 müssen sich aber nicht gegenüberliegen, sondern können am Gerät jede geeignete Position einnehmen. Über den Atemgaseingang 2 kann ein Atemgas in den Atemgasweg 3 eingespeist werden. Über den Atemgasausgang 4 kann ein Atemgasgas aus dem Atemgasweg entlassen werden.

Im oder am Atemgasweg 3 kann, wie schematisch dargestellt, mindestens ein Gebläse 5 angeordnet sein, der den Atemgasweg 3 in eine Saugseite 6 und eine Druckseite 7 unterteilt. Das Gebläse 5 kann als Axialgebläse oder vorzugsweise, wie gezeigt, als Radialgebläse ausgebildet sein. Die Saugseite 6 ist zwischen dem Atemgaseingang 2 und einem Gebläseeingang 8 ausgebildet. Der Gebläseeingang 8 kann als Saugstutzen ausgebildet sein. Die Druckseite 7 ist zwischen einem Gebläseausgang 9 und dem Atemgasausgang 4 ausgebildet. Der Gebläseausgang 9 kann als Druckstutzen ausgebildet sein. Das Gebläse 5 kann aufgrund seiner Geometrie eine erste akustische Impedanz 14 aufweisen. Das Beatmungsgerät 1 umfasst ferner eine Vorrichtung 10 zur Schalldämpfung. Die Vorrichtung 10 kann aufgrund seiner Geometrie zumindest eine weitere akustische Impedanz 15,16,17 aufweisen, die von der ersten akustischen Impedanz 14 abweicht.

Die Vorrichtung 10 ist druckseitig 7 im Atemgasweg 3 angeordnet. Alternativ oder zusätzlich kann die Vorrichtung 10 auch saugseitig 6 im Atemgasweg 3 angeordnet sein. Die Vorrichtung 10 kann einen ersten Anschluss 30 und/oder einen zweiten Anschluss 31 umfassen, um die Vorrichtung 10 an den Atemgasweg 3 eines Beatmungsgerätes 1 anzuschließen. Die Vorrichtung 10 bildet sodann zumindest bereichsweise den Atemgasweg 3 aus, vorzugsweise den Atemgasweg 3 auf der Druckseite 7.

Der erste Anschluss 30 kann hierbei einen mittelbaren oder unmittelbaren Anschluss der Vorrichtung 10 an das Gebläse 5 ermöglichen. Es kann wie gezeigt zwischen dem Druckstutzen 9 des Gebläses 5 und dem ersten Anschluss 30 der Vorrichtung 10 eine verbindende Leitung 3,7 geben, so dass die Vorrichtung 10 mittelbar an das Gebläse 3 angeschlossen ist. In manchen Ausführungsformen kann der Druckstutzen 9 auch direkt in oder an dem ersten Anschluss 30 münden, so dass die Vorrichtung 10 unmittelbar an das Gebläse 5 anschließt. Sodann kann das Atemgas vom Druckstutzen 9 aus direkt die Vorrichtung 10 eintreten (nicht gezeigt).

Der zweite Anschluss 31 kann einen mittelbaren oder unmittelbaren Anschluss der Vorrichtung 10 an den Atemgasausgang 4 des Beatmungsgerätes 1 ermöglichen. Es kann wie gezeigt zwischen dem zweiten Anschluss 31 der Vorrichtung 10 und dem Atemgasausgang 4 eine verbindende Leitung 3,7 geben, so dass die Vorrichtung 10 mittelbar an den Atemgasausgang 4 angeschlossen ist. In manchen Ausführungsformen kann der zweite Anschluss 31 auch direkt in den Atemgasausgang 4 münden, so dass die Vorrichtung 10 unmittelbar an den Atemgasausgang 4 anschließt. Sodann kann das Atemgas von der Vorrichtung 10 aus direkt aus dem Beatmungsgerät 1 austreten (nicht gezeigt).

Die Vorrichtung 10 umfasst eine Kammer 11, die zwischen dem Gebläseausgang 9 und dem Atemgasausgang 4 angeordnet ist. Die Kammer 11 kann eine zweite akustische Impedanz 15 aufweisen. Die geometrische Form und der Querschnitt der Kammer 11 ist eine andere als die des Gebläses 5. Somit kann die erste akustische Impedanz 14 derart von der zweiten akustischen Impedanz 15 abweichen, dass ein durch das Gebläse 5 erzeugter Schall gedämpft wird.

Ferner umfasst die Vorrichtung 10 einen Kanal 13, der zwischen der Kammer 11 und dem Atemgasausgang 4 angeordnet ist. Der Kanal 13 weist einen geringeren Durchmesser als die Kammer 11 auf. Der Kanal 13 kann eine dritte akustische Impedanz 16 aufweisen. Da die geometrische Form und der Querschnitt der Kanals 13 eine andere ist als die der Kammer 11 und/oder des Gebläses 5, weicht die dritte akustische Impedanz 16 von der ersten akustischen Impedanz 14 und/oder der zweiten akustischen Impedanz 15 ab, so dass ein durch das Gebläse 5 erzeugter Schall zusätzlich gedämpft wird.

Das Atemgas kann innerhalb des Beatmungsgerätes 1 folgendem Atemgasweg 3 folgen: Das Atemgas kann über den Atemgaseingang 2 in den Atemgasweg 3 eintreten. Vom Atemgaseingang 2 gelangt das Atemgas in die Saugseite 6 des Atemgasweges 3 und über den Saugstutzen 8 in das Gebläse 5. Das Gebläse 5 liefert die Förderenergie des Atemgases und befördert das Atemgas über den Druckstutzen 9 in die Druckseite 7 des Atemgasweges 3. Dort tritt das Atemgas in die Vorrichtung 10 ein. Zunächst gelangt das Atemgas in die Kammer 11 und durchströmt diese. Von der Kammer 11 tritt das Atemgas in den Kanal 13 ein und durchströmt diesen in seiner Längsrichtung. Vom Kanal 13 kann das Atemgas aus der Vorrichtung 10 austreten und über den Atemgasausgang 4 aus dem Beatmungsgerät 1 heraus und zu einem nicht gezeigten Patienten befördert werden.

Beim Übergang vom Gebläse 5 zur Vorrichtung 10, genauer gesagt vom Gebläse 5 zur Kammer 11, verändert sich die Geometrie und/oder der Querschnitt des Atemgasweges 3 vorzugsweise sprunghaft. Beim Übergang von der Kammer 11 zum Kanal 13 verändert sich abermals die Geometrie und/oder der Querschnitt des Atemgasweges 3 vorzugsweise sprunghaft. Dadurch treten Schallreflexionen auf, so dass der durch das Beatmungsgerät 1 erzeugte Schall gedämpft wird. Das Verhältnis des Volumens des Kanals 13 zum Volumen der Kammer 11 kann zu diesem Zwecke mindestens 1:2 betragen, bevorzugt mindestens 1:4, besonders bevorzugt 1:10.

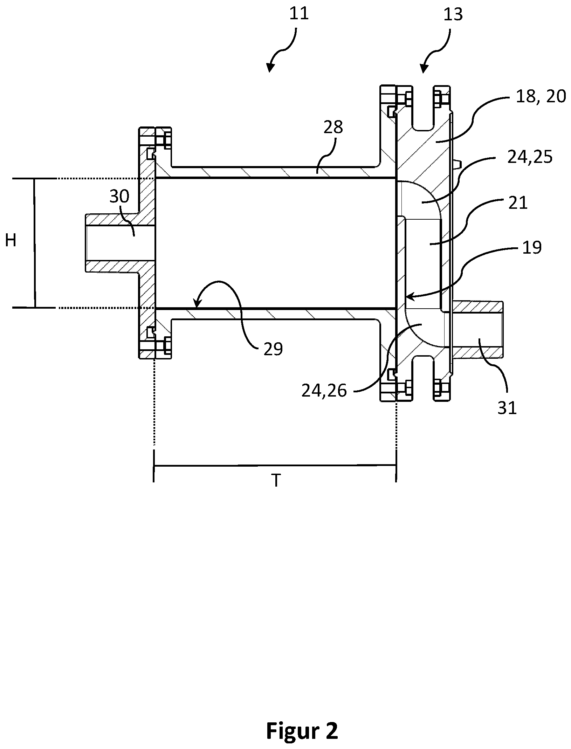

**Figur 2** zeigt die Vorrichtung 10 gemäß einer ersten Ausführungsform der Erfindung. Die Vorrichtung 10 umfasst die Kammer 11 und den Kanal 13. In Figur 2 ist gezeigt, dass die Vorrichtung einen modularen Aufbau aufweisen kann. Ein Modul kann dabei einem Abschnitt 11, 12 der Vorrichtung 10 entsprechen. Ein erstes Modul kann die Kammer 11 umfassen, ein weiteres den Kanal 13. Die Module können hierbei als Stecksystem ausgebildet sein, so dass die Reihenfolge der Module veränderbar ausgebildet sein kann. Es können in manchen Ausführungsformen auch mehrere Kammern 11 und Kanäle 13 hintereinander angeordnet sein.

Aus Fig. 2 wird ersichtlich, dass die Kammer 11 eine Kammerwand 28 mit einer ein Lumen der Kammer 11 umschließenden Kammer-Innenwandung 29 umfassen kann. Die Kammer-Innenwandung 29 ist vorzugsweise konstant ausgebildet. Die Kammer-Innenwandung 29 kann ohne Brüche, Ecken, Kanten, Lücken oder dergleichen gleichmäßig ausgebildet sein. Vorzugsweise ist die Kammer-Innenwandung 29 ebenmäßig und glatt ausgebildet. Dadurch kann eine besonders ebenmäßige, vorzugsweise laminare Strömung ohne Turbulenzen ermöglicht werden. Somit herrscht in der Kammer 11 ein gleichmäßiges Strömungsmuster, was sich positiv auf einen Druckaufbau des Atemgases auswirken kann.

Das Atemgas kann über den ersten Anschluss 30 in das Lumen der Kammer 11 gelangen und diese durchströmen. Die Kammer 11 weist eine Tiefe T und eine Höhe H und eine hier nicht darstellbare Breite auf. Die Tiefe T der Kammer 11 sollte nicht zu gering sein, da sonst die einkommende Strömung als Freistrahl direkt auf die Gegenwand treffen würde. Die Gegenwand kann vorzugsweise eine glatte Wand ohne Kanten oder ähnliches sein um Strömungsgeräusche zu vermeiden.

Aus Figur 2 ist weiterhin ersichtlich, dass der Kanal 13 eine Kanalwand 18 mit einer ein Lumen des Kanals 13 umschließenden Innenwandung 19 umfasst. Die Innenwandung 19 ist vorzugsweise konstant ausgebildet ist. Der Kanal 13 kann in seiner Längsrichtung betrachtet zumindest teilweise gerade ausgebildet sein. Der Kanal 13 kann zumindest teilweise auch gebogen ausgebildet sein. Die Vorrichtung 10 kann somit einen Kanal 13 umfassen, der mindestens einen geraden Abschnitt 21,22 und mindestens einen gebogenen Abschnitt 25, 26 umfasst. Der gebogene Abschnitt 25,26 weist hierfür mindestens eine Biegung 24 auf, so dass der Kanal 13 in seiner Längsrichtung gebogen ausgebildet ist.

In dem Ausführungsbeispiel gemäß Fig. 2 weist der Kanal 13 einen ersten gebogenen Abschnitt 25 und einen zweiten gebogenen Abschnitt 26 auf sowie einen den ersten gebogenen Abschnitt 25 mit dem zweiten gebogenen Abschnitt 26 verbindenden geraden Abschnitt 21. Diese Ausführungsform bietet den Vorteil einer besonderen Platzersparnis. Durch die beiden gebogenen Abschnitte 25,26 erfährt das Atemgas darüber hinaus eine Umlenkung, wodurch Schall zusätzlich unterdrückt werden kann. Die Biegung 24 kann wie gezeigt beispielsweise rechtwinklig ausgebildet sein. Die Biegung 24 kann somit ausgebildet sein, das Atemgas in den gebogenen Abschnitten 25,26 um jeweils 90° umzulenken. Andere Winkel der Biegungen 24 sind denkbar.

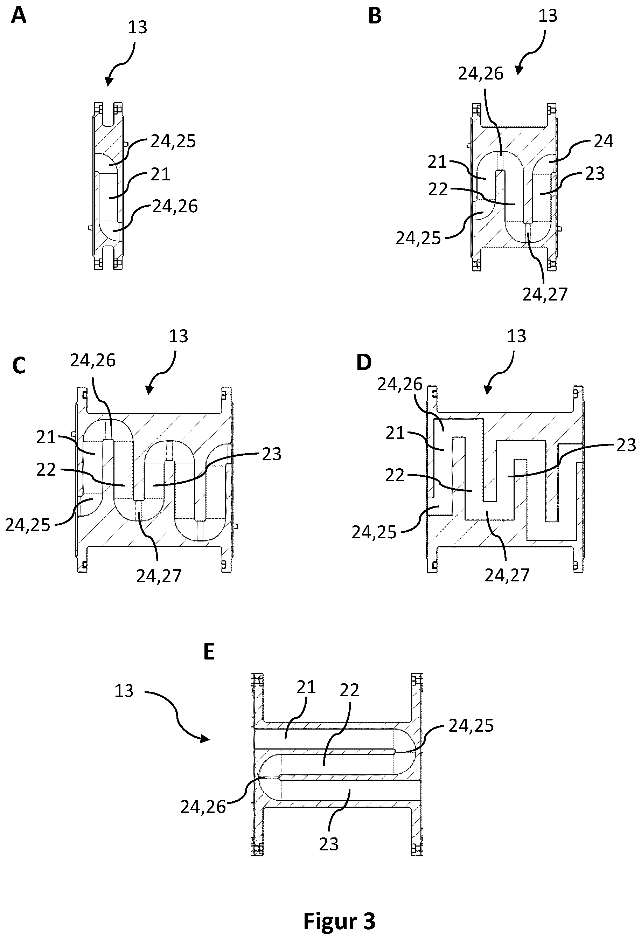

**Figur 3** zeigt beispielhafte Ausführungsformen eines Kanal 13 gemäß der Erfindung. Aus Figur 3 ist ersichtlich, dass der Kanal 13 bezüglich seiner Längsrichtung die unterschiedlichsten Formen aufweisen kann. Der Kanal 13 kann in seiner einfachsten Ausführungsform gerade ausgebildet sein (nicht gezeigt).

Der Kanal 13 kann vorzugsweise mindestens eine Biegung 24 aufweisen wie in den Figuren 3A-E gezeigt. Die Biegung 24 kann rund (Fig. 3A-C, 3E) oder eckig (Fig. 3D) ausgebildet sein.

Beispielsweise kann der Kanal 13 an mindestens einer Stelle eine U-förmige Biegung aufweisen, die den Kanal um 180° abbiegt. Der Kanal 13 kann auch an mindestens einer Stelle eine L-förmige Biegung aufweisen, die den Kanal um 90° abbiegt. Die Biegung(en) können rund oder eckig ausgebildet sein.

Über die Biegung 24 kann grundsätzlich jegliche Abwinkelung des Kanals 13 in einem 2- oder 3-dimensionalen Raum realisiert sein. In bevorzugten Ausführungsformen kann der Winkel der Biegung 24 jedoch mindestens 45° betragen. Die Biegung 24 kann den Kanal 13 in seiner Längsrichtung bevorzugt zwischen 45° und 180° abbiegen.

**Fig. 3A** zeigt eine Ausführungsform des Kanals 13 mit einem ersten gebogenen Abschnitt 25 und einem zweiten gebogenen Abschnitt 26. Die beiden gebogenen Abschnitte 25,26 sind über einen geraden Abschnitt 21 miteinander verbunden. In dieser konkreten Ausführungsform weisen der erste gebogene Abschnitt 25 und der zweite gebogene Abschnitt jeweils eine L-förmige Biegung 24 auf, die 90° beträgt. Die beiden Biegungen 24 sind hierbei entgegengesetzt ausgebildet.

**Fig. 3B** zeigt eine Ausführungsform des Kanals 13 mit vier gebogenen Abschnitten 25,26,27..., die jeweils über gerade Abschnitte 21,22,23 miteinander verbunden sein können. Diese Ausführungsform weist beispielhaft drei gerade Abschnitte 21,22,23 auf. Die geraden Abschnitte 21,22,23 können in ihrer Längsrichtung gleichlang ausbildet sein und/oder unterschiedliche Längen aufweisen.

In dieser konkreten Ausführungsform weisen der erste gebogene Abschnitt 25 und der vierte, letzte gebogene Abschnitt jeweils eine L-förmige Biegung 24 auf, die 90° beträgt. Der zweite gebogene Abschnitt 26 und der dritte gebogene Abschnitt 27 weisen jeweils eine U-förmige Biegung 24 auf, die 180° beträgt. Durch die derart ausgestalteten Biegungen 24 können die Längsachsen der geraden Abschnitte 21,22,23 jeweils parallel zueinander angeordnet sein. Dies bietet den Vorteil einer besonderen Platzersparnis.

**Fig. 3C und 3D** zeigen eine Ausführungsformen des Kanals 13 mit einer Mehrzahl an gebogenen Abschnitten 25,26,27... und einer Mehrzahl an geraden Abschnitten 21,22,23... Der Kanal 13 weist somit in seiner Längsrichtung einen mäanderförmig gewundenen Verlauf auf. Die Längsachsen der geraden Abschnitte 21,22,23 können dabei wie gezeigt parallel zueinander angeordnet sein. Eine andere Anordnung ist denkbar.

Die Anzahl an geraden und gebogenen Abschnitten kann variabel gewählt sein. Dabei gilt, dass je mehr Umlenkungen das Atemgas erfährt, desto schallärmer kann die Vorrichtung 10 ausgebildet sein. Mehrfache Umlenkungen des Atemgases können jedoch auch in einen Druckverlust resultieren. Somit sollten bei der Ausgestaltung des Kanals 13 die Vorteile und die Nachteile der Umlenkungen miteinander abgewogen werden. Die hier gezeigten Ausführungsbeispiele haben sich als ideale Ausführungen erwiesen, die eine optimale Schallunterdrückung bei ausreichendem Druckaufbau gewährleisten.

**Fig. 3E** zeigt eine Ausführungsform des Kanals 13 mit zwei gebogenen Abschnitten 25,26. Die gebogenen Abschnitte 25,26 weisen eine U-förmige Biegung 24 auf. Diese Ausführungsform weist zudem drei gerade Abschnitte 21,22,23 auf. Die gebogenen Abschnitte 25,26 sind jeweils über die geraden Abschnitte 21,22,23 miteinander verbunden.

In alternativen Ausführungsformen kann der Kanal 13 auch vollständig gebogen ausgebildet sein und beispielsweise spiralförmig aufgewickelt vorliegen (nicht gezeigt). In noch weitern nicht gezeigten Ausführungsformen kann auch eine beliebige Anordnung aus 2- und 3-dimensionalen Biegungen 24 vorliegen, so dass eine symmetrische oder asymmetrische Anordnung des Kanals 13 realisiert ist.

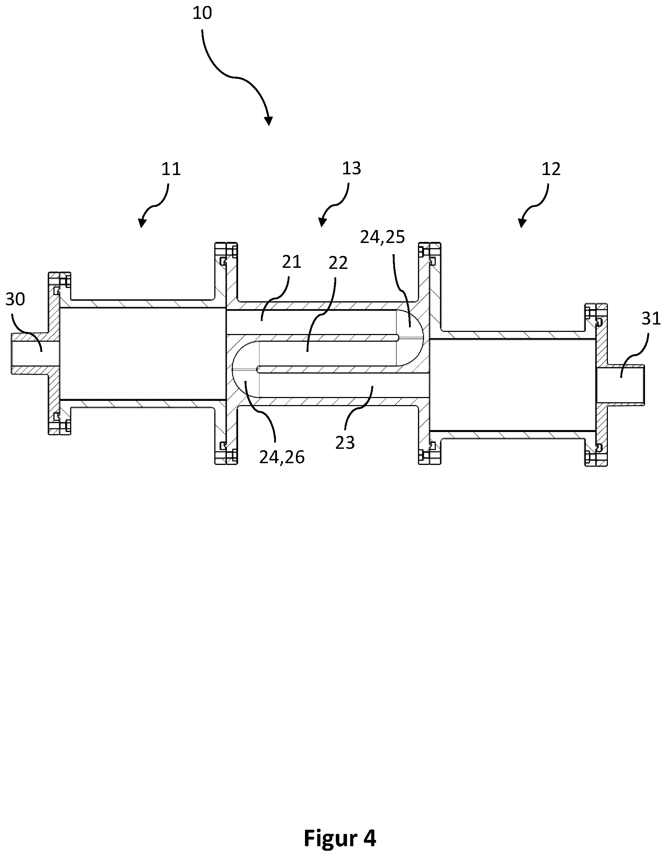

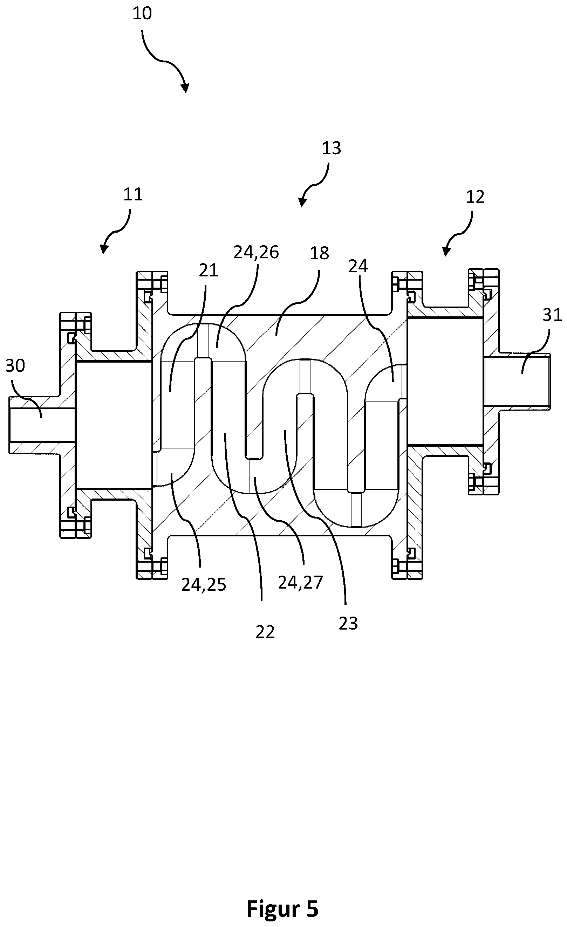

**Figur 4** zeigt die Vorrichtung 10 gemäß einer zweiten Ausführungsform der Erfindung und **Figur 5** zeigt die Vorrichtung 10 gemäß einer dritten Ausführungsform der Erfindung. Es ist gezeigt, dass die Vorrichtung 10 neben der Kammer 11 und dem Kanal 13 eine zweite Kammer 12 umfassen kann. Die zweite Kammer 12 ist vorzugsweise zwischen dem Kanal 13 und dem Atemgasausgang 4 angeordnet. Die zweite Kammer 12 kann eine vierte akustische Impedanz 17 aufweisen, die von der ersten akustischen Impedanz 14 und/oder der zweiten akustischen Impedanz 15 und/oder der dritten akustischen Impedanz 16 abweicht. Dadurch kann ein durch das Gebläse 5 erzeugter Schall zusätzlich gedämpft werden.

Die Kammer 11 und die zweite Kammer 12 können identisch dimensioniert sein und identische geometrische Formen aufweisen wie in den Figuren 4 und 5 exemplarisch gezeigt. In manchen Ausführungsformen kann es jedoch vorteilhaft sein, dass die Kammer 11 und die zweite Kammer 12 unterschiedliche Dimensionen und/oder geometrische Formen aufweisen (nicht gezeigt). Dadurch kann gewährleistet sein, dass die zweite akustischen Impedanz 15 (der Kammer) und die vierte akustische Impedanz 17 (der zweiten Kammer) voneinander abweichen.

Die Kammer 11 und/oder die zweite Kammer 12 kann ein Befeuchter sein oder einen Befeuchter umfassen (nicht gezeigt). Der Befeuchter kann eingerichtet sein, um das Atemgas mit einer Flüssigkeit zu befeuchten und/oder zu erwärmen.

In Figur 4 ist gezeigt, dass der Kanal 13 einen ersten geraden Abschnitt 21, einen zweiten geraden Abschnitt 22 und einen dritten geraden Abschnitt 23 umfassen kann. Ferner umfasst der Kanal 13 einen ersten gebogenen Abschnitt 25, der den ersten geraden Abschnitt 21 mit dem zweiten geraden Abschnitt 22 fluidisch verbindet. Zudem kann der Kanal 13 einen zweiten gebogenen Abschnitt 26 umfassen, der den zweiten geraden Abschnitt 22 mit dem dritten geraden Abschnitt 23 fluidisch verbindet. Dabei können die gebogenen Abschnitte 25,26 wie gezeigt derartige Biegungen 24 aufweisen, dass die Längsachse des ersten geraden Abschnitts 21 parallel zur Längsachse des zweiten geraden Abschnitts 22 ist und/oder die Längsachse des zweiten geraden Abschnittes 22 parallel zur Längsachse des dritten geraden Abschnitts 23 ist.

Der Kanal 13 kann somit in seiner Längsrichtung mindestens einmal U-förmig gebogen sein (nicht gezeigt), bevorzugt mindestens zweimal wie in Fig. 4 gezeigt. In weiter bevorzugten Ausführungsformen kann der Kanal auch mindestens viermal U-förmig gebogen vorliegen oder noch häufiger (nicht gezeigt).

**In** **Figur 5** ist gezeigt, dass der Kanal 13 eine Mehrzahl an geraden Abschnitten 21,22,23... und gebogenen Abschnitten 25,26,27... umfasst, so dass der Kanal 13 in seiner Längsrichtung einen mäanderförmig gewundenen Verlauf aufweist. Die geraden Abschnitte 21,22,23... können wie gezeigt parallel verlaufende Längsachsen aufweisen. Die Biegungen 24 der gebogenen Abschnitte 25,26,27... können gleiche und/oder unterschiedliche Winkel aufweisen. In dem konkreten Ausführungsbeispiel gemäß Fig. 5 weisen der erste gebogene Abschnitt 24 und der letzte gebogene Abschnitt jeweils eine L-förmige Biegung 24 um 90° auf. Die übrigen gebogenen Abschnitte 26,27... weisen jeweils U-förmige Biegungen auf, so dass die Längsachsen der geraden Abschnitte 21,22,23 parallel zueinander sind.

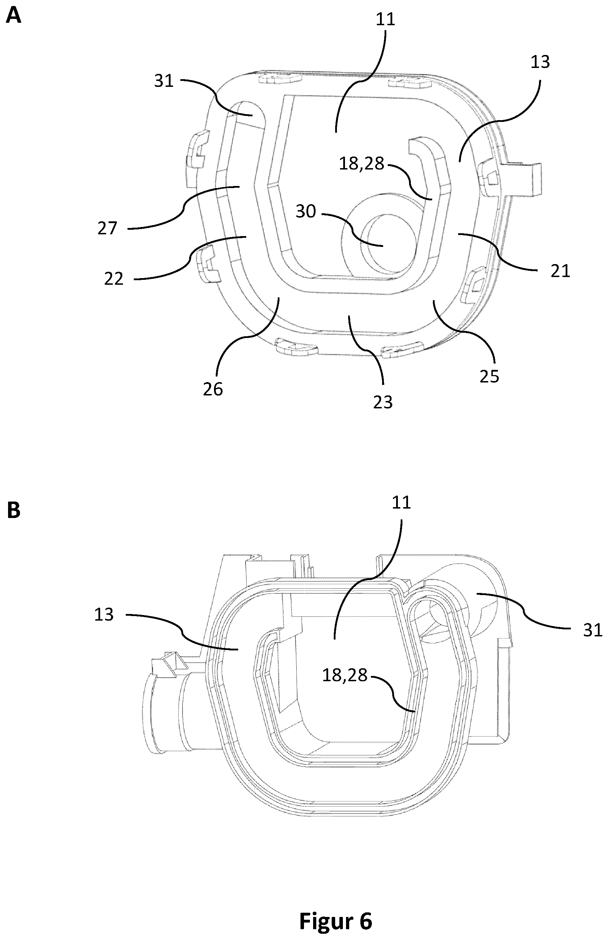

**Figur 6** zeigt die Vorrichtung 10 gemäß einer vierten Ausführungsform der Erfindung, wobei die Ansichten in Fig. 6A und 6B die jeweilige Innenansicht nach einem Längsschnitt durch die Vorrichtung 10 zeigen.

Aus diesem konkreten Ausführungsbeispiel wird ersichtlich, dass der Kanal 13 eine Mehrzahl an geraden Abschnitten 21,22,23... und gebogenen Abschnitten 25,26,27... umfassen kann. Aus dieser Abbildung wird ferner ersichtlich, dass der Kanal 13 und die Kammer 11 zumindest bereichsweise durch dieselbe Wand 18, 28 ausgebildet sein können. Der Kanal 13 kann derart angeordnet, dass dieser zumindest teilweise um die erste Kammer 11 herum ausgebildet ist und das Lumen der Kammer 11 zumindest teilweise umschließt. Der Kanal 13 kann derart gebogen ausgebildet sein, dass er insgesamt U-Förmig um die Kammer 11 herum angeordnet ist. In alternativen, hier nicht gezeigten Ausführungsformen kann auch vorgesehen sein, dass der Kanal nur gebogene Abschnitte umfasst, so dass dieser oval, kreisrund oder spiralförmig ausgebildet ist und beispielsweise um die Kammer herumgewickelt ausgebildet ist. Dies bietet den Vorteil einer besonders platzsparenden Ausführung.

Das Atemgas kann über den Anschluss 30 in die Kammer 11 geleitet werden. Aus dieser kann das Atemgas in den Kanal 13 geleitet werden, wobei das Atemgas um 90° abgelenkt werden kann. Durch die sprunghafte Verkleinerung bzw. Vergrößerung des Querschnitts des Atemgasweges vom Anschluss 30 zur Kammer 11 sowie von der Kammer 11 zum Kanal 13 kann eine effektive Schalldämpfung stattfinden. Auch die Umlenkung des Atemgases kann schalldämpfend wirken.

Das Atemgas kann vom Kanal 13 direkt zum Anschluss 31 geleitet werden. Es ist auch denkbar, dass nach dem Kanal 13 eine zweite Kammer 12 angeordnet ist (hier nicht dargestellt). Diese kann in manchen Ausführungsformen in einem Befeuchter angeordnet sein. In den Figuren 2, 4, 5 und 6 sind jeweils Ausführungsformen gezeigt, bei denen zwischen der Kammer und dem Kanal und/oder zwischen dem Kanal und der zweiten Kammer jeweils Übergänge bestehen, die als einfache Öffnung in der Wandung ausgebildet sind. Die Elemente Kammer, Kanal, zweite Kammer grenzen aneinander an und das Atemgas kann von einem Element ins Nächste strömen. In alternativen Ausführungsformen ist es auch denkbar, dass ein Element in das nächste hineinragend ausgebildet ist. Dann kann der Kanal in die Wandung hinein oder durch die Wandung der Kammer bzw. der zweiten Kammer hindurchgeführt sein und in die Kammer hineinragen (nicht gezeigt).

Die Vorrichtung kann wie gezeigt die Anschlüsse 30, 31 umfassen, um die Vorrichtung an den Atemgasweg 3 eines Beatmungsgerätes anzuschließen. Die Vorrichtung kann über den ersten Anschluss stromaufwärts mittelbar oder unmittelbar an den Druckstutzen des Gebläses anschließen. Die Vorrichtung kann über den zweiten Anschluss stromabwärts stromaufwärts mittelbar oder unmittelbar an den Atemgasausgang des Beatmungsgerätes anschließen. Erster Anschluss und/oder zweiter Anschluss können wie gezeigt als Stutzen ausgebildet sein, an die die jeweiligen Komponenten des Beatmungsgerätes angeschlossen werden können. In manchen Ausführungsformen können die Anschlüsse auch als einfache Öffnung ausgebildet sein (nicht gezeigt). So kann beispielsweise der Druckstutzen direkt an eine Öffnung in der Kammer-Wandung angrenzen oder in die Wandung hinein oder durch die Wandung der Kammer hindurchgeführt sein. In manchen Ausführungsformen kann der Druckstutzen zumindest teilweise in die Kammer hineinragen. Ferner kann der Kanal oder alternativ die zweite Kammer eine Öffnung aufweisen, die mittelbar oder unmittelbar an den Atemgasausgang des Beatmungsgerätes anschließt bzw. diesen ausbildet.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

- 1: Beatmungsgerät
- 2: Atemgaseingang
- 3: Atemgasweg
- 4: Atemgasausgang
- 5: Gebläse (Atemgasantrieb)
- 6: Saugseite
- 7: Druckseite
- 8: Gebläseeingang (Saugstutzen)
- 9: Gebläseausgang (Druckstutzen)
- 10: Vorrichtung zur Schalldämpfung
- 11: Kammer
- 12: Zweite Kammer
- 13: Kanal
- 14: erste akustische Impedanz
- 15: zweite akustische Impedanz
- 16: dritte akustische Impedanz
- 17: vierte akustische Impedanz
- 18: Kanalwand
- 19: Innenwandung
- 20: Material
- 21: erster gerader Abschnitt
- 22: zweiter gerader Abschnitt
- 23: dritter gerader Abschnitt
- 24: Biegung
- 25: erster gebogener Abschnitt
- 26: zweiter gebogener Abschnitt
- 27: dritter gebogener Abschnitt
- 28: Kammerwand
- 29: Kammer-Innenwandung
- 30: erster Anschluss
- 31: zweiter Anschluss
- 32: Gehäuse

## Patentansprüche

1. Vorrichtung (10) zur Schalldämpfung für ein Beatmungsgerät (1) wobei das Beatmungsgerät (1) neben der Vorrichtung (10) einen Atemgaseingang (2), einen Atemgasausgang (4) und einen zwischen dem Atemgaseingang (2) und dem Atemgasausgang (4) ausgebildeten Atemgasweg (3) mit einem Gebläse (5) umfasst, wobei das Gebläse (5) ausgebildet ist, um ein Atemgas vom Atemgaseingang (2) zum Atemgasausgang (4) zu fördern, so dass der Atemgasweg (3) eine Saugseite (6) und eine Druckseite (7) aufweist, wobei die Vorrichtung (10) umfasst:
- eine Kammer (11), die ausgebildet ist, um zwischen dem Gebläse (5) und dem Atemgasausgang (4) auf der Druckseite (7) im Atemgasweg (3) angeordnet zu werden,
- einen rohrförmigen Kanal (13), der ausgebildet ist, um die Kammer (11) fluidisch mit dem Atemgasausgang (4) zu verbinden.

2. Vorrichtung (10) nach Anspruch 1, wobei das Gebläse (5) eine erste akustische Impedanz (14) aufweist und wobei die Kammer (11) eine zweite akustische Impedanz (15) aufweist und wobei der Kanal (13) eine dritte akustische Impedanz (16) aufweist, wobei die akustischen Impedanzen (14, 15, 16) derart voneinander abweichen, dass ein durch das Gebläse (5) erzeugter Schall gedämpft wird.

3. Vorrichtung (10) nach einem der vorhergehenden Ansprüche, wobei der Kanal (13) in seiner Längsrichtung betrachtet zumindest teilweise gerade und/oder zumindest teilweise gebogen ausgebildet ist.

4. Vorrichtung (10) nach einem der vorhergehenden Ansprüche, wobei der Kanal (13) mindestens einen geraden Abschnitt (21,22,23...) und mindestens einen gebogenen Abschnitt (25,26,27...) umfasst, wobei der gebogene Abschnitt (25,26,27...) mindestens eine Biegung (24) aufweist, so dass der Kanal (13) in seiner Längsrichtung gebogen ist.

5. Vorrichtung (10) nach Anspruch 4, wobei die Biegung (24) rund oder eckig ausgebildet ist und/oder dass die Biegung (24) mindestens 10° beträgt, bevorzugt mindestens 45°, besonders bevorzugt zwischen 45° und 180°.

6. Vorrichtung (10) nach einem der vorhergehenden Ansprüche, wobei der Kanal (13) in seiner Längsrichtung an mindestens einer Stelle U-förmig und/oder L-förmig gebogen ist, bevorzugt an mindestens zwei Stellen.

7. Vorrichtung (10) nach einem der vorhergehenden Ansprüche, wobei der Kanal (13) einen ersten geraden Abschnitt (21), einen zweiten geraden Abschnitt (22) und einen den ersten geraden Abschnitt (21) mit dem zweiten geraden Abschnitt (22) verbindenden gebogenen Abschnitt (25) umfasst, wobei der gebogene Abschnitt (25) eine U-förmige Biegung (24) aufweist, so dass die Längsachsen des ersten geraden Abschnitts (21) und des zweiten geraden Abschnitts (22) parallel zueinander sind.

8. Vorrichtung (10) nach einem der vorhergehenden Ansprüche, wobei der Kanal (13) einen ersten gebogenen Abschnitt (25) und einen zweiten gebogenen Abschnitt (26) und einen den ersten gebogenen Abschnitt (25) mit dem zweiten gebogenen Abschnitt (26) verbindenden geraden Abschnitt (21) umfasst.

9. Vorrichtung (10) nach einem der vorhergehenden Ansprüche, wobei der Kanal (13) eine Mehrzahl an geraden Abschnitten (21,22,23...) und gebogenen Abschnitten (25,26,27...) umfasst, so dass der Kanal (13) in seiner Längsrichtung zumindest abschnittsweise einen mäanderförmigen Verlauf aufweist.

10. Vorrichtung (10) nach einem Anspruch 8, wobei der Kanal (13) eine Mehrzahl an geraden Abschnitten (21,22,23...) und gebogenen Abschnitten (25,26,27...) umfasst und derart angeordnet ist, dass der Kanal (13) zumindest teilweise die erste Kammer (11) ausbildet, indem eine Wand (18) des Kanals zumindest bereichsweise eine Wand (28) der Kammer ausbildet.

11. Vorrichtung (10) nach einem Anspruch 10, wobei der Kanal (13) um die Kammer (11) herum U-förmig angeordnet ist.

12. Vorrichtung (10) nach einem der vorhergehenden Ansprüche, wobei die Vorrichtung (10) eine zweite Kammer (12) umfasst, die zwischen dem Kanal (13) und dem Atemgasausgang (4) angeordnet ist.

13. Vorrichtung (10) nach einem der vorhergehenden Ansprüche, wobei die zweite Kammer (12) eine vierte akustische Impedanz (17) aufweist, die von der ersten akustischen Impedanz (14) und/oder der zweiten akustischen Impedanz (15) und/oder der dritten akustischen Impedanz (16) abweicht, sodass ein durch das Gebläse (5) erzeugter Schall zusätzlich gedämpft wird.

14. Vorrichtung (10) nach einem der vorhergehenden Ansprüche, wobei der Kanal (13) und/oder die Kammer (11) und/oder die zweite Kammer (12) als Hohlraum in einem nicht-porösem Material ausgebildet ist, insbesondere in einen nicht porösen Kunststoff.

15. Vorrichtung (10) nach einem der vorhergehenden Ansprüche, wobei der Kanal (13) ein Volumen von 100 cm³ - 10.000 cm³ aufweist, bevorzugt von 500 cm³ - 5.000 cm³, besonders bevorzugt von 800 - 4.500 cm³.

16. Vorrichtung (10) nach einem der vorhergehenden Ansprüche, wobei der Kanal (13) eine Länge von 1 mm - 1.000 mm aufweist, bevorzugt 10 mm - 500 mm, besonders bevorzugt 10 - 350 mm und/oder einen Durchmesser von1 mm - 30 mm, bevorzugt 5 mm - 20 mm, besonders bevorzugt 10 - 15 mm.

17. Vorrichtung (10) nach einem der vorhergehenden Ansprüche, wobei die Kammer (11) und/oder die zweite Kammer (12) ein Volumen von 2.000 cm³ - 40.000 cm³ aufweist, bevorzugt von 4.000 cm³ - 20.000 cm³, besonders bevorzugt von 10.000 - 15.000 cm³.

18. Vorrichtung (10) nach einem der vorhergehenden Ansprüche, wobei die Kammer (11) und/oder die zweite Kammer (12) eine Tiefe (T) von 50 mm - 120 mm aufweist, bevorzugt von 80 mm - 100 mm und/oder eine Höhe (H) von 10 mm - 70 mm, bevorzugt von 20 mm - 40 mm und/oder eine Breite von 10 mm - 100 mm, bevorzugt von 40 mm - 60 mm.

19. Vorrichtung (10) nach einem der vorhergehenden Ansprüche, wobei die Kammer (11) und/oder die zweite Kammer (12) und/oder der Kanal (13) unterschiedlich dimensioniert sind, so dass die akustischen Impedanzen (15,16,17) voneinander abweichen.

20. Vorrichtung (10) nach einem der vorhergehenden Ansprüche, wobei die Vorrichtung (10) einen ersten Anschluss (30) und/oder einen zweiten Anschluss (31) umfasst, um die Vorrichtung (10) an den Atemgasweg (3) eines Beatmungsgerätes (1) anzuschließen.

21. Beatmungsgerät (1) mit einem Atemgaseingang (2), einem Atemgasausgang (4) und einem zwischen dem Atemgaseingang (2) und dem Atemgasausgang (4) ausgebildeten Atemgasweg (3) mit einem Gebläse (5), das ausgebildet ist, um ein Atemgas vom Atemgaseingang (2) zum Atemgasausgang (4) zu fördern, so dass der Atemgasweg (3) eine Saugseite (6) und eine Druckseite (7) aufweist, umfassend eine Vorrichtung (10) nach einem der vorhergehenden Ansprüche, wobei die Vorrichtung (10) in dem Atemgasweg (3) zwischen dem Gebläse (5) und dem Atemgasausgang (4) angeordnet ist und über den ersten Anschluss (30) und den zweiten Anschluss (31) derart an den Atemgasweg (3) angeschlossen ist, dass die Kammer (11) zwischen dem Gebläse (5) und dem Atemgasausgang (4) auf der Druckseite (7) im Atemgasweg (3) angeordnet ist, und dass der rohrförmige Kanal (13) die Kammer (11) fluidisch mit dem Atemgasausgang (4) verbindet.

22. Beatmungsgerät (1) nach Anspruch 21, wobei das Beatmungsgerät (1) ferner einen Befeuchter zum Befeuchten und/oder Erwärmen des Atemgases umfasst, wobei der Befeuchter die Kammer (11) und/oder die zweite Kammer (12) und/oder den Kanal (13) umfasst.
